(19) Europäisches Patentamt
European Patent Office
Office européen des brevets

(11) **EP 1 533 740 A2**

(12) **EUROPEAN PATENT APPLICATION**

(43) Date of publication:
25.05.2005 Bulletin 2005/21

(51) Int Cl.7: **G06F 19/00**

(21) Application number: 04026222.2

(22) Date of filing: 04.11.2004

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: 14.11.2003 JP 2003384980

(71) Applicant: **Feel Fine Kabushiki Kaisha Tokyo 100-0005 (JP)**

(72) Inventors:
• **Hayashi, Junichi**
  **Itabashi-ku Tokyo, 173-0013 (JP)**
• **Tanahashi, Norie Feel Fine Kabushiki Kaisha Tokyo 100-0005 (JP)**
• **Terabe, Masahiro Mitsubishi Research Inst., Inc. Tokyo 100-8141 (JP)**
• **Kondou, Takashi Mitsubishi Research Inst., Inc. Tokyo 100-8141 (JP)**
• **Tanimoto, Hiroshi Feel Fine Kabushiki Kaisha Tokyo 100-0005 (JP)**

(74) Representative: **Viering, Jentschura & Partner Steinsdorfstrasse 6 80538 München (DE)**

(54) **Age assessment apparatus and age assessment method**

(57) An age assessment apparatus capable of assessing a substantial age of an examinee from various active degrees is provided. The age assessment apparatus scores detailed test items Z1jk with respect to kinesthetic activity of an examinee in accordance with kinesthetic performance scoring criteria and calculates kinesthetic age difference value $\Delta y1j$ which expresses a degree of age divergence from an actual age, scores detailed test items Z2jk with respect to mental activity in accordance with mental state scoring criteria and calculates mental age difference value $\Delta y2j$, scores detailed test items Z3jk with respect to physical activity in accordance with physical information scoring criteria and calculates physical age difference value $\Delta y3j$, then calculates a substantial age FF of the examinee by subtracting a sum obtained by multiplying each of the age difference values and each of weight coefficients pi which constitute an eigenvector which is a solution of an eigenvalue problem in analytic hierarchy process together from the actual age "a" of the examinee.

Fig. 1

**EP 1 533 740 A2**

## Description

FIELD OF THE INVENTION

The present invention relates to an age assessment apparatus and an age assessment method, and in particular relates to an age assessment apparatus and an age assessment method that assess a substantial age of an examinee based upon biological information of the examinee.

DESCRIPTOR OF THE RELATED ART

Health control for each person has lately drawn attention with the coming of an aging society. Those who take care of health, for example, not only enter the hospital or the like for a periodic medical examination, but also give a consideration to health such as avoiding an unbalanced diet or a high calorie intake and taking exercise at a fitness club or the like in a daily life. Thus, an average person having little medical knowledge can pay attention to daily health control. However, it is difficult to evaluate own health degree objectively.

In order to solve this problem, for example, JPA 11-318830 discloses a health status measuring instrument which gives a questionnaire about smoking, drinking and the like to an examinee, measures biological (physical) information on blood pressure, pulse rate, etc. , of the examinee, and judges a health status based upon the results of the questionnaire and the biological information. Further, JPA 2002-063278 discloses a health degree evaluation system that visibly displays a health degree of an individual from the past to the future by indexing of comparison between a health standard model and various disease models and evaluating the health degree when health affecting elements such as smoking, drinking and the like are varied as parameters.

Furthermore, a technique that analyzes maximum and minimum points of pulse wave detected from an examinee to calculate a vascular estimate age of the examinee is disclosed (ex. JPA 2000-051166). Besides, a technique that calculates a substantial age of an external aspect (appearance) by indexing the form of wrinkles of forehead and crow's feet is also disclosed (ex. JPA 2002-330943).

In the techniques of JPA 11-318830 and JPA 2002-063278, since the health degree (health status) is calculated by supplementing biological information of the examinee with health affecting elements such as smoking, drinking and the like, they are available to prevent the examinees from having specific diseases. However, they have difficulty in judging a comprehensive health degree from many sides such as kinesthetic performance (ability) and a mental state that healthy examinees have. While, in the techniques of JPA 2000-051166 and JPA 2002-063278, it is easy for an average person who has little medical knowledge to understand his/her own health degree in that they calculate the substantial age. However, it is difficult to judge a comprehensive health degree of the examinee since they estimate the substantial age of the examinee only by the health degree of specific portions of the examinee.

## SUMMARY OF THE INVENTION

Accordingly, it is preferable to judge a total health degree of the examinee from many aspects and to give the examinee understandable information. If, for example, a kinesthetic active degree (kinesthetic performance information) and a mental active degree (mental state information) are added, other than a physical active degree, as indicators for a health degree of the examinee, and if those indicators are evaluated uniformly to express his/her substantial age, it would be easy for the examinee, whosoever he/she has some disease, has its tendency or has good health, to understand his/her health degree on the basis of a difference between his/her actual age and substantial age.

In view of the above circumstances, an object of the present invention is to provide an age assessment apparatus and an age assessment method capable of assessing a substantial age of an examinee from various active degrees.

In order to achieve the above object, a first aspect of the present invention is directed to an age assessment apparatus that assesses a substantial age of an examinee based upon biological information of the examinee, comprising: a first calculator for scoring a plurality of kinesthetic performance information of the examinee in accordance with predetermined kinesthetic performance scoring criteria and calculating a kinesthetic age difference value which expresses a degree of age divergence from an actual age of the examinee with respect to kinesthetic activity which expresses an active degree of a kinesthetic performance of the examinee; a second calculator for scoring a plurality of mental state information of the examinee in accordance with predetermined mental state scoring criteria and calculating a mental age difference value which expresses a degree of age divergence from an actual age of the examinee with respect to mental activity which expresses an active degree of a mental state of the examinee; a third calculator for scoring a plurality of physical information of the examinee in accordance with predetermined physical information scoring criteria and calculating a physical age difference value which expresses a degree of age divergence from an actual age of the examinee with respect to physical activity which expresses an active degree of a body of the examinee;

EP 1 533 740 A2

and an age calculator for calculating a substantial age of the examinee, in which the kinesthetic activity, the mental activity and the physical activity are integrated, based upon an actual age of the examinee and each of the age difference values calculated by the first to third calculators.

In the first aspect, a plurality of kinesthetic performance information cf the examinee is scored in accordance wirth predetermined kinesthetic performance scoring criteria and a kinesthetic age difference value which expresses a degree of age divergence from an actual age of the examinee with respect to kinesthetic activity which expresses an active degree of a kinesthetic performance of the examinee is calculated by the first calculator, a plurality of mental state information of the examinee is scored in accordance with predetermined mental state scoring criteria and a mental age difference value which expresses a degree of age divergence from an actual age of the examinee with respect to mental activity which expresses an active degree of a mental state of the examinee is calculated by the second calculator, a plurality of physical information of the examinee is scored in accordance withpredetermined physical information scoring criteria and a physical age difference value which expresses a degree of age divergence from an actual age of the examinee with respect to physical activity which expresses an active degree of a body of the examinee is calculated by the third calculator, and then, by the age calculator, a substantial age of the examinee, in which the kinesthetic activity, the mental activity and the physical activity are integrated, is calculated based upon an actual age of the examinee and each of the age difference values calculated by the first to third calculators. According to the first aspect, since the substantial age of the examinee, in which the kinesthetic activity, the mental activity and the physical activity are integrated, is calculated based upon the actual age of the examinee and each of the age difference values calculated by the first to third calculators, it is possible to assess the substantial age from various active degrees of the examinee.

In the first aspect, at least one of the kinesthetic performance scoring criteria. the mental state scoring criteria and the physical information scoring criteria may be criteria classified by a male and a female, and an age or an age group, and the kinesthetic performance scoring criteria, the mental state scoring criteria and the physical information scoring criteria may be scoring criteria corresponding to a distribution scope of statistical distribution of a large number of examinees. The age calculator may add/subtract a sum obtained by multiplying each of the age difference values calculated by the first to third calculators and each of weight coefficients corresponding to the each of the age difference values together to/from the actual age of the examinee to calculate the substantial age of the examinee. At this time, it is preferable that the weight coefficients constitute an eigenvector which is a solution of an eigenvalue problem including a pair comparison matrix obtained by analytic hierarchy process, and a sum of the each of the weight coefficients can be 1. Further, the first to third calculators score the plurality of kinesthetic performance information in accordance with the kinesthetic performance scoring criteria, the plurality of mental state information in accordance with the mental state scoring criteria, and the plurality of physical information in accordance with the physical information scoring criteriabypoints, respectively, then, may convert the scored points to the age difference values which express the degrees of the age divergence from the actual ages of the examinee with respect to the plurality of kinesthetic performance information, the plurality of mental state information and the plurality of physical information respectively, and may calculate sums obtained by multiplying each of the age difference values and each of weight coefficients which correspond to the each of the age difference values and which are solutions of eigenvalue problems including pair comparison matrixes obtained by analytic hierarchy process together as the kinesthetic age difference value, the mental age difference value and the physical age difference value, respectively. In the present aspect, it is preferable that the physical information includes at least three pieces of information among information as to blood, information as to body composition including obesity index, information as to bone density, information as to vascular sclerosis and information as to equilibrium function of the examinee, and that the kinesthetic performance information includes at least three pieces of information among information as to muscular power, information as to muscular endurance strength, information as to instantaneous power, information as to flexibility and information as to quickness of the examinee, and that the mental state information includes at least three pieces of information as to vitality, information as to tranquilizing status, information as to beta-endorphin in blood, information as to adaptability in chaotic analysis and resistance in chaotic analysis of the examinee.

Further, in order to achieve the above object, a second aspect of the present invention is directed to an age assessment method for assessing a substantial age of an examinee based upon biological information of the examinee, comprising: a first calculating step for scoring a plurality of kinesthetic performance information of the examinee in accordance with predetermined kinesthetic performance scoring criteria and calculating a kinesthetic age difference value which expresses a degree of age divergence from an actual age of the examinee with respect to kinesthetic activity which expresses an active degree of a kinesthetic performance of the examinee; a second calculating step for scoring a plurality of mental state information of the examinee in accordance with predetermined mental state scoring criteria and calculating a mental age difference value which expresses a degree of age divergence from an actual age of the examinee with respect to mental activity which expresses an active degree of a mental state of the examinee; a third calculating step for scoring a plurality of physical information of the examinee in accordance with predetermined physical information scoring criteria and calculating a physical age difference value which expresses a degree of age

3

divergence from an actual age of the examinee with respect to physical activity which expresses an active degree of a body of the examinee; and an age calculating step for calculating a substantial age of the examinee, in which the kinesthetic activity, the mental activity and the physical activity are integrated, based upon an actual age of the examinee and each of the age difference values calculated by the first to third calculating steps.

BRIEF DESCRIPTION OF THE DRAWINGS

Fig. 1 is a block diagram, showing an age assessment system of an embodiment to which the present invention is applicable;

Fig. 2 is an explanatory diagram illustratively showing an assessment concept of a substantial age according to the age assessment system of the embodiment;

Fig. 3 is an explanatory diagram illustratively showing a calculation concept of a periphery area according to a centroid fluctuation measuring instrument;

Fig. 4 is an explanatory diagram illustratively showing a testing concept according to an integration method based on a statistical average value;

Fig. 5A is an explanatory diagram illustratively showing a testing concept according to an integration method based on a statistical distribution;

Fig. 5B is an explanatory diagram illustratively showing a testing concept according to an integration method based on a hypothetical distribution; and

Fig. 6 is a flowchart of an age assessment routine executed by a CPU of a WWW server at a FF information providing site.

DETAILED DESCRIPTION OF THE PREFERRED EMBODIMENTS

With reference to the drawings, an embodiment of an age assessment system which receives information about an examinee from a client through an Internet, evaluates a substantial age of the examinee, and transmits the evaluation (assessed results) to the client and to which the present invention is applied will be explained below.

(Structure)

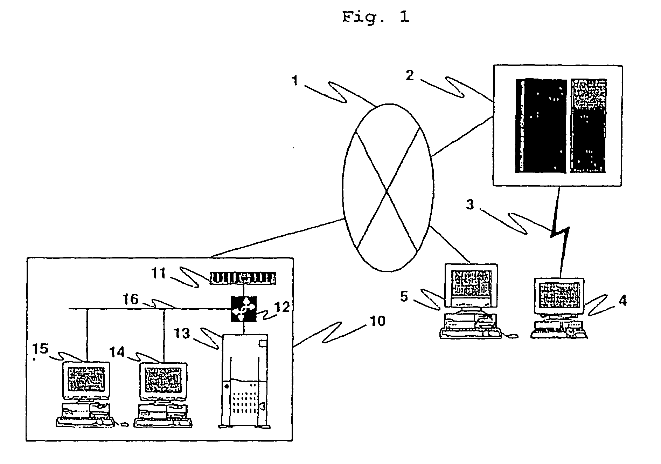

As shown in Fig. 1, a provider site 2 is connected to a network 1. The provider site 2 is constituted, for example, by a server such as a WWW server, a FTP server, or the like and a plurality of computers which controls and updates the server. A terminal 4 such as a personal computer and the like can establish a link with the provider site 2 through a public line 3.A terminal 5 such as a business computer and the like is also connected to the network 1.

Further, a FF information providing site 10 for providing (transmitting) information on a substantial age of an examinee (hereinafter called "FF information") to the terminals 4, 5 is connected to the network 1. The FF information providing site 10 has a firewall 11 for arming against unfair access from an outside network. The network 1 is connected to a bus typed LAN 16 which is extended inside the FF information providing site 10 via the firewall 11 and a router 12. A WWW server 13 which serves as a part of an age assessment apparatus and which transmits the FF information to the terminals 4, 5 is connected to the LAN 16. A database (DB) server 14 serving as a part of an age assessment apparatus, a charging (accounting) computer 15 which calculates charging fees for providing the FF information per client, and unillustrated various computers are also connected to the LAN 16. Incidentally, the WWW server 13 mainly serves as a first to third calculators and an age calculator of the present invention, and the database server 14 serves as a part of each of the first to third calculators.

An age assessment program for assessing or evaluating a substantial age FF of an examinee based upon kinesthetic performance information, mental state information and physical information (detailed test items Z stated below) as well as an actual age "a" of the examinee, all of which are received from the clients, namely, terminals 4, 5 is implanted to the WWW server 13. Here, the actual age "a" is defined as an actual calendar age of the examinee, and the substantial age FF is defined as an age of the examinee which is assessed (calculated) by the age assessment program of the present embodiment.

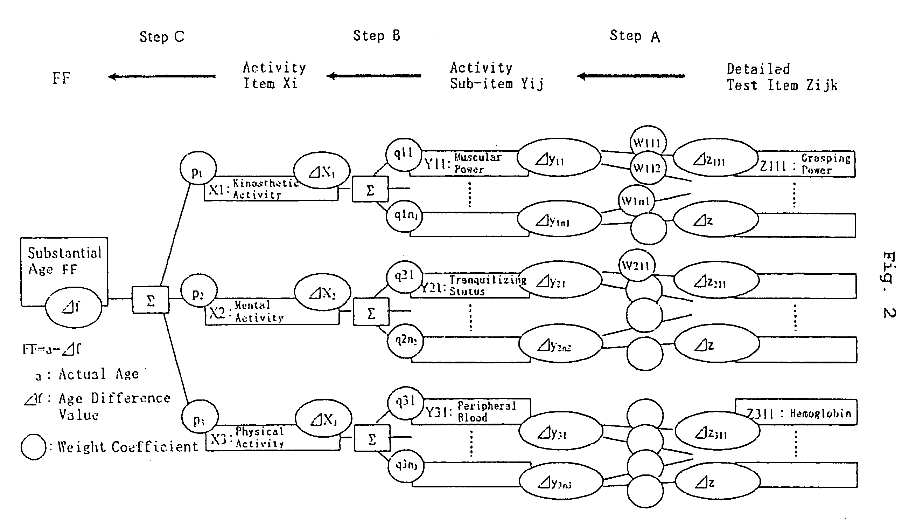

Fig. 2 illustratively shows an assessment concept of the substantial age FF according to the age assessment program. The concept for assessing the substantial age FF can be briefed in that activity sub-items Y are calculated through an integration step A from detailed test items Z at every kinesthetic activity which expresses an active degree of kinestheticperformance of the examinee, mental activity which expresses an active degree of a mental state of the examinee and physical activity which expresses an active degree of a body of the examinee, then, activity items X with respect to each of the kinesthetic, mental, and physical activities are calculated from the activity sub-items Y through an integration step B, and in an integration step C, age difference values which express age divergences from actual ages of the examinee with respect to each of the activity items X are calculated, then, a sum $\Delta f$ obtained by multiplying each of the age difference values and each of weight coefficients p together is subtracted from the actual age "a" to calculate the substantial age FF. However, the details are as follows:

As shown in Fig. 2, the activity items Xi (i=1 to 3) are constituted by three of kinesthetic activity X1, mental activity X2 and physical activity X3. Every activity item Xi has a tree structure with a parent-children relationship, activity sub-items Yij being children, and detailed test items Zijk being children thereof. Namely, the activity items Xi have tree structures classified and determined by a plurality of physicians specialized in preventive medicine and psychic medicine (hereafter called "specialists") in view of a relationship, similarity, many-sided evaluative characteristic of information of the activity sub-items Yij in order to obtain the kinesthetic activity X1, mental activity X2 and physical activity X3. The activity sub-items Yij also have tree structures classified and determined by the specialists in view of a relationship, similarity and many-sided evaluative characteristic of information on the detailed test items Zijk. Each of the detailed test items Zijk belong to any one of the activity sub-items Yij, and each of the activity sub-items Yij belong to any one of the activity items Xi. Incidentally, "j" expresses the number of the activity sub-item belonging to the activity items Xi, and "k" expresses the number of the detailed test item belonging to the activity sub-items Yij. The following Tables 1 to 3 show relationships between the activity items Xi, the activity sub-items Yij and the detailed test items Zijk with respect to the kinesthetic activity XI, the mental activity X2, and the physical activity x3 of the present embodiment.

Table 1

| Activity Item X | Activity Sub-item Y | Detailed Test Item Z | Remarks Remarks |
|---|---|---|---|
| Kinesthetic Activity (X1) | Muscular Power | Grasping Power (Z111-2) | kg |
| | (Y11) | Strength of Back Muscle (Z113) | kg |
| | Muscular Endurance Strength (Y12) | Raising of Upper Body (Z121) | cm |
| | Instantaneous Power (Y13) | Vertical Jump (Z131-2) | cm |
| | Flexibility (Y14) | Overstay and Slouch (Z141-2) | cm |
| | Quickness (Y15) | Repetition of Sideway Jumps (Z151) | times |

Table 2

| Activity Item X | Activity Sub-item Y | Detailed Test Item Z | Remarks |
|---|---|---|---|
| Mental Activity (X2) | Tranquilizing Status (Y21) | Answers 1-20 (Z211-30) | Questionnaire |
| | Vitality (Y23) | Answers 1-15 (Z231-45) | Questionnaire |
| | Happiness (Y25) | Beta-endorphin (Z251) | pg/ml |
| | Adaptability (Y26) | Adaptability in Chaotic Analysis (Z261) | - |
| | Resistance (Y27) | Resistance in Chaotic Analysis (Z271) | - |

Table 3

| Activity Item X | Activity Sub-item Y | Detailed Test Item Z | Remarks | |
|---|---|---|---|---|
| Physical Activity (X3) | Peripherel Blood (Y31) | Hemoglobin (Z311) | 13-5-17.5 11.5-15.0 | g/dl |
| | | Hematocrit (Z312) | 39.7-45.0 34.8-45.0 | % |
| | | Erythrocyte (Z313) | 430.0-570.0 380.0-500.0 | X 10,000 /µl |
| | | Leukocyte (Z314) | 3300-9000 3300-9000 | /µl |
| | | Blood Platelet (Z315) | 14.0-34.0 14.0-34.0 | X 10,000 µl |
| | Lipids (Y32) | Total Cholestarol (Z321) | 120.0-219.0 120.0-219.0 | mg/dl |
| | | HDL Cholesterol (Z322) | 40.0-70.0 45.0-75.0 | mg/dl |
| | | LDL Cholesterol (Z323) | 65.0-139.0 65.0-139.0 | mg/dl |
| | | Neutral Fats (Z324) | 30.0-149.0 30.0-149.0 | mg/dl |
| | Liver Function (Y33) | GOT (Z331) | 10.0-40.0 10.0-40.0 | IU/l/ 37 deg.C |
| | | GPT (Z332) | 5.0-45.0 5.0-45.0 | IU/l/ 37 deg. C |
| | | Gamma GTP (Z333) | 0.0-80.0 0.0-80.0 | IU/l/ 37 deg. C |
| | Gout (Y34) Gout (Y34) | Uric Acid (Z341) | 3.8-7.5 2.4-5.8 | mg/dl |
| | Diabetes (Y35) | GHblc (Z351) | 4.3-5.8 4.3-5.8 | % |
| | Body Body.. Composition (Y36) (Y36) (Y36) | Percent Body Fat (Z361) | 17-23 | % |
| | | Obesity Index (Z362) | (-10)-10 | % |
| | Bone Density <Y37) | Distal Bone Density (Z371) | 6-stage evaluation | % % |
| | Vascular Sclerosis (Y38) | baPWV (Z381-4) | 5-stage evaluation | sigma (σ) |
| | Equilibrium (Function (Y39) | Periphery Area (Z391) | 4-stage evaluation | $cm^2$ |

As shown in Table 1, five activity sub-items, muscular power (Y11) , muscular endurance strength (Y12), instantaneous power (Y13), flexibility (Y14) and quickness (Y15), belong to activity item X1. Table 1 shows that, for example, two detailed test items, grasping power (Z111-2) and strength of backmuscle (Z113) belong to an activity sub-item, muscular power (Y11), and one detailed test item, raising of upper body (Z121) belongs to an activity sub-item, muscular endurance strength (Y12) . Incidentally, in the detailed test items in Table 1, grasping power (Z111-2) expresses right hand grasping power (Z111) and left hand grasping power (Z112). Further, vertical jump (Z131-2) as well as overstay and slouch (Z141-2) express first vertical jump (Z131), second vertical jump (Z132), first overstay and slouch (Z141) and second overstay and slouch, respectively.

As shown in Table 2, five activity sub-items, tranquilizing status (Y21), vitality (Y23), happiness (Y25), adaptability (Y26) and resistance (Y27), belong to an activity item, mental activity X2.

Answers to a questionnaire consisting of 20 questions belong to tranquilizing status (Y21) as detailed test items Z211-Z230, and answers to a questionnaire consisting of 15 questions belong to vitality (Y23) as detailed test items Z231-Z245. Tables 4 and 5 respectively show an example for numerically converting the answers to the tranquilizing status (Y21) and the vitality (Y23). Withrespect to each of the detailed test items Z211-Z230, the examinee selects one of answering numbers 1-4 corresponding to "rare", "seldom", "often", "always" as an answer, and with respect to each of the detailed test items Z231-Z245, he/she selects either of an answering number 1 or 2 corresponding to "yes" or "no" as an answer.

Table 4

| No. | Contents of Questionnaire | Answer | | | | Remarks |
|---|---|---|---|---|---|---|
| | | rare | seldom | often | always | |
| 1 | Do you feel good? | 1 | 2 | 3 | 4 | |
| 2 | Do you tend to get tired? | 1 | 2 | 3 | 4 | reverse |
| 3 | Do you feel very sad? | 1 | 2 | 3 | 4 | reverse |
| | | | | | | |
| 20 | Do you proceed with things steadily ? 1 | 2 | 3 | 4 | | |

Table 5

| No. | Contents of Questionnaire | Answer | | Remarks |
|---|---|---|---|---|
| | | yes | no | |
| 1 | Are you satisfied with the present life? | 1 | 2 | |
| 2 | Do you often feel bored? | 1 | 2 | reverse |
| 3 | Do you feel that you are full of vitality? | 1 | 2 | |
| | | | | |
| 20 | Do you feel that you are forgetful than others? | 1 | 2 | |

A detailed test item, an amount (picogram/milliliter) of beta-endorphin (Z251) inblood, belongs to happiness (Y25). Further, a detailed test item, adaptability in chaotic analysis (Z261) belongs to adaptability (Y26), and resistance in chaotic analysis (Z271) belongs to resistance (Y27), respectively.

The chaotic analysis is to measure adaptability and resistance as mental activity of the examinee based on biological data which are obtained from pulse waves of the examinee. The adaptability in chaotic analysis (Z261) and the resistance in chaotic analysis (Z271) can be measured by a chaotic analysis instrument such as brand name "KOKORODA" which is introduced to the market by Kabushiki Kaisha Computer. Convenience, and the like. In the chaotic analysis instrument, the sampling period of the pulse wave(s) is set to 0.005 seconds, for example. By repeating calculation of Lyapunov exponents with the pulse wave of a predetermined period (ex. sampling numbers of 3500) to plot the indexes, and, shifting the period a little (ex. sampling numbers of 200) to calculate Lyapunov exponents again, Lyapunov exponents can be calculated continuously to obtain chaos attractor. The adaptability is calculated by quantifying, with the Lyapunov exponents, chaotic information according to the chaos attractor of the pulse wave. In a state that the examinee had a good sleep, the adaptability decreases and the examinee has a mentally relaxed state. On the other hand, the resistance can be calculated by squaring (namely power of) the amplitude of the pulse wave. The resistance means strength for battling against invasion and destruction from the outside, and it also includes immune strength. In a state that the examinee had a good sleep, energy is consumed by negative defense and the power increases with discharging of calorie. It is known that a limit of power is automatically coordinated as organism since the power which became too large wi th passage of time declines once due to a coordinating point. Incidentally, the adaptability (Z261) and the resistance (Z271) in chaotic analysis do not have a unit system.

As shown in Table 3, other than one group of activity sub-items consisting of peripheral blood (Y31), lipids (Y32), liver function (Y33), gout (Y34) and diabetes (Y35), which are obtained from the blood of the examinee, five activity sub-items in total, including body composition (Y36) , bone density (Y37), vascular sclerosis (Y38) and equilibrium function (Y39), belong to physical activity X3.

Five detailed test items, hemoglobin (Z311), hematocrit (Z312), erythrocyte (Z313), leukocyte (Z314) and blood platelet (Z315), belong to the peripheral blood (Y31). Four detailed test items, total cholesterol (Z321), HDL cholesterol (Z322), LDL cholesterol (Z323) and neutral fats (Z324) belong to the lipids (Y32). Three detailed test items Z331-Z333, GOT, GPT and gamma GTP, belong to the liver function (Y33). One detailed test item, uric acid (Z341), GHblc (Z351) belongs to the gout (Y34), diabetes (Y35), respectively.

Two detailed test item, percent body fat (Z361) and obesity index (degree of obesity) (Z362) belong to the body composition (Y36). Distal bone density (Z371) measured by a DEXA type osteo-densitometer belongs to the bone density (Y37). The unit systemof the bone density is $g/cm^2$ primarily, however, because such type of osteo-densitometer has a standard value according to statistical data, it can output a percentage of the distal bone density of the examinee by setting the standard value to 100%.

Four detailed test items, baFWV (Z381-Z384) expressing elasticity of artery in a right arm, a left arm, a right foot and a left foot, belong to the vascular sclerosis. Incidentally, in the present embodiment, a vascular sclerosis measuring apparatus, which has a standard value of the healthy subject and which can output deviation σ for the examinee from the standard value, is used for measuring the detailed test items baPWV (Z381-Z334).

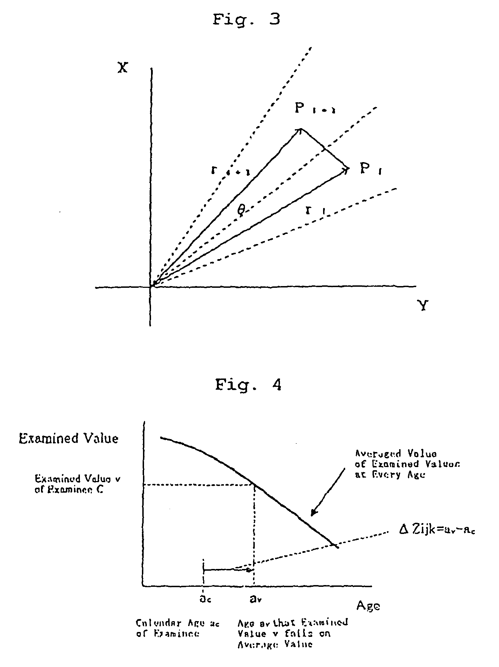

Periphery area (Z391) measured by a centroid fluctuation measuring instrument, as a detailed test item, belongs to the equilibrium function (Y39). The centroid fluctuation measuring instrument measures positions of centers of gravity for a predetermined time (ex. 60 seconds), and it can obtain the periphery area by integrating envelope areas which are divided by a predetermined angle and which are surrounded with outermost lines of centroid fluctuation trajectory. Namely, as shown in Fig. 3, a periphery of the origin of the centroid fluctuation is divided, for example, into 120 equal parts (3 degrees). The point of the center of gravity, whose radius takes the maximum value in each of the divided ranges, is found along the whole divided ranges, then an area Si ($Si = ri \times (ri + 1) \times$ sine $\theta$ /2; here, $\theta$=3 degrees in a case of 120 equal parts) of an triangle constituted by linking adjacent maximum radii and the original is found, and an integral value I shown in Formula (1) with respect to the periphery of the origin is calculated to obtain the periphery area ($cm^2$). Incidentally, this kind of centroid fluctuation measuring instrument is also on the market, and, for example , brand name GRAVICODER (modal numbpr : GS3000)of Anima Kabushiki Kaisha may be listed as one of such instruments.

$$ I \;\simeq\; \sum_{i=1}^{120} S_i \qquad\qquad \bullet\;\bullet\;\bullet \qquad (1) $$

Accordingly, the fundamental structure of the age assessment program is that five activity sub-items Yij belong to each of the three activity items Xi, the kinesthetic activity X1, the mental activity X2 and the physical activity X3, and that one or more detailed test items Zijk belong to each of the activity sub-items-Yij.

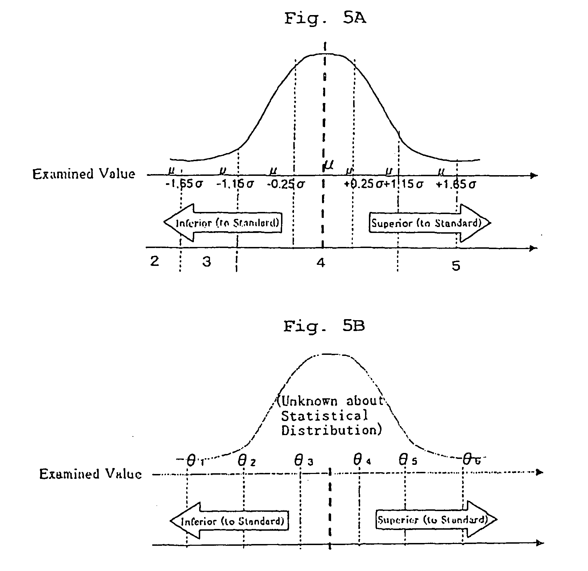

In the age assessment program, each of the detailed test items Zijk is scored in general with 6 stages of 0-5 (point) in accordance with scoring criteria. For example, as shown in Fig. 5A, in a case that a population of some detailed test item Zijk has a normal distribution, if the deviation of the detailed test item Zijk of the examinee is +1.15 sigma or more, then the scored point of 5 is given, if the deviation is +1.15 sigma to -1.15 sigma, then the scored point of 4 is given, if the deviation is -1.15 sigma to -1.65 sigma, then the scored point of 3 is given, if the deviation is -1.65 sigma to -2 sigma, then the scored point of 2 is given, if the deviation is -2 sigma to -3 sigma, then the scored point of 1 is given, and if the deviation is -3 sigma or less, then the scored point of 0 is given. Incidentally, the threshold values for the scoring criteria were decided by studying of the plurality of specialists.

Tables 6A and 6B below show the scoring criteria of the detailed test items Zljk which belong to the kinesthetic activity X1, which was prepared in the above manner. In scoring the detailed test item Zljk, an arithmetic mean is carried out for the right hand grasping power (Z111) and the left hand grasping power (Z112) to obtain the grasping power (Z111-2) (grasping power = (Z111 + Z112)/2), then the grasping power is scored in accordance with the scoring criteria. In the same manner, each of arithmetic mean is carried out for both the first vertical jump (Z131), the second vertical jump (Z132), and the first overstay and slouch (Z141), the second overstay and slouch (Z142). For example, in a case that the examinee is a male in fifties, his right hand grasping power (Z111) is 41.5 kg, and his left hand grasping power (Z112) is 37.5 kg, since an arithmetic mean thereof becomes 39.5 kg, the scored point "4" is obtained from Table 6.

Table 6A

| Item | Age Group | Male | Female | Scored Point |
|---|---|---|---|---|
| Grasping Power | twenties | 50 or more | 30 or more | S |
| | | 48 or more | 28 or more | 4 |
| | | 46 or more | 26 or more | 3 |
| | | 44 or more | 24 or more | 2 |
| | | 42 or more | 22 or more | 1 |
| | | less than 42 | less than 22 | 0 |
| | ... | | | |
| | fifties | 40 or more | 25 or more | 5 |
| | | 38 or more | 23 or more | 4 |
| | | 36 or more | 21 or more | 3 |
| | | 34 or more | 19 or more | 2 |
| | | 32 or more | 17 or more | 1 |
| | | less than 32 | less than 17 | 0 |
| Strength of Back Muscle | twenties | 145 or more | 80 or more | 5 |
| | | 140 or more | 75 or more | 1 |
| | | 135 or more | 70 or more | 3 |
| | | 130 or more | 65 or more | 2 |
| | | 125 or more | 60 or more | 1 |
| | | less than 125 | less than 60 | 0 |
| | ... | | | |
| | fifties | 110 or more | 60 or more | 5 |
| | | 105 or more | 55 or more | 4 |
| | | 100 or more | 50 or more | 3 |
| | | 95 or more | 45 or more | 2 |
| | | 90 or more | 40 or more | 1 |
| | | less than 90 | less than 40 | 0 |

Table 6A   (continued)

| Item | Age Group | Male | Female | Scored Point |
|---|---|---|---|---|
| Raising of Upper Body | twenties | 30 or more | 20 or more | 5 |
| | | 25 or more | 18 or more | 4 |
| | | 20 or more | 16 or more | 3 3 |
| | | 15 or more | 14 or more | 2 |
| | | 10 or more | 12 or more | 1 |
| | | less than 10 | less than 12 | 0 |
| | ... | | | |
| | fifties | 10 or more | 5 or wore | 5 |
| | | 9 or more | 4 or more | 4 |
| | | 6 or more | 3 or more | 3 |
| | | 4 or more | 2 or more | 2 |
| | | 2 or more | 1 or more | 1 |
| | | less than 2 | less than 1 | 0 |

Table 6B

| Item | Age | Male | Female | | Score Point |
|---|---|---|---|---|---|
| Vertical Jump | twenties | 55 or more | 35 or more | | 5 |
| | | 53 or more | 33 or more | | 4 |
| | | 51 or more | 31 or more | | 3 |
| | | 49 or more | 29 or more | | 2 |
| | | 47 or more | 27 or more | | 1 |
| | | less than 47 | less than 27 | | 0 |
| | ... | | | | |
| | fifties fifties | 35 or more | 20 or more | | 5 |
| | | 33 or more | 18 or more | | 4 |
| | | 31 or more | 16 or more | | 3 |
| | | 29 or mere | 14 or more | | 2 |
| | | 27 or more | 12 or more | | 1 |
| | | less than 27 | less than 12 | | 0 |

Table 6B   (continued)

| Item | Age | Male | Female | | Score Point |
|---|---|---|---|---|---|
| Overstay and Slouch | twenties | 56 or more | 56 or more | | 5 |
| | | 47 or more | 48 or more | | 4 |
| | | 38 or more | 40 or more | | 3 |
| | | 27 or more | 31 or more | | 2 |
| | | 20 or more | 24 or more | | 1 |
| | | less than 20 | less than 24 | | 0 |
| | | ... | | | |
| | fifties | 56 or more | 56 or more | | 5 |
| | | 47 or more | 48 or more | | 4 |
| | | 38 or more | or more | | 3 |
| | | 27 or more | 31 or more | | 2 |
| | | 20 or more | 24 or more | | 1 |
| | | less than 20 | less than 24 | | 0 |
| Repetition of Sideway Jumps | twenties | 45 or more | 40 or more | | 5 |
| | | 43 or more | 33 or more | | 4 |
| | | 41 or more | 36 or more | | 3 |
| | | 39 or more | 34 or more | | 2 |
| | | 32 or more | 32 or more | | 1 |
| | | less than 32 | less than 32 | | 0 |
| | | ... | | | |
| | fifties | 30 or more | 30 or more | | 5 |
| | | 26 or more | 28 or more | | 4 |
| | | 26 or more | 26 or more | | 3 |
| | | 24 or more | 24 or more | | 2 |
| | | 22 or more | 22 or more | | 1 |
| | | less than 22 | less than 22 | | 0 |

With respect to the answers 1-20 (Z211-Z220) to the questionnaire of the detailed test items which belong to the tranquilizing status (Y21), a total value is calculated as whole answers, then, the total value is scored in accordance with the scoring criteria shown Table 7 below Namely, in principle, numeric conversion is carried out by the answering number as it is. However, regarding the answer listed as "reverse" in the remarks column of Table 4, numeric conversion is carried out by (5 - (answering number) } . Then, a total value is calculated by summing each of the numerically converted numbers with respect to the answers 1-20. For example, in a case that the examinee selects the answer "seldom" to Question 1: "Do you feel good?" of the detailed test item (Z211), "2" is numerically converted, and in a case that the examinee selects the answer "rare" to Question 3 (Z213): "Do you feel very sad?", 5 - 1 = 4 is numerically converted since "reverse" is listed in the remarks column. If the total value of the numerically converted numbers is, for example, 65, the scored point "4" is obtained from Table 7.

Table 7

| Activity Sub-item | Total Value | Scored Point |
|---|---|---|
| Tranquilizing Status (Y21) | 70 or more | 5 |
| | 60 or more | 4 |
| | 50 or more | 3 |
| | 40 or more | 2 |
| | 30 or more | 1 |
| | less than 30 | 0 |
| Vitality (Y23) | 13 or more | 5 |
| | 12-9 | 4 |
| | 8-7 | 3 |
| | 6-4 | 2 |
| | 3-2 | 1 |
| | less than 1 | 0 |
| Happiness (Y25) | More than 150% | 5 |
| | 150%-50% | 4 |
| | 50%-40% | 3 |
| | 40%-30% | 2 |
| | 30%-20% | 1 |
| | less than 20% | 0 |
| Adaptability (Y26) | (-0.1)-0.1 | 5 |
| | 0.1-0.15 (-0.15) - (-0.1) | 4 |
| | 0.15-0.25 (-0.25)-(-0.15) | 3 |
| | 0-25-0.3 (-0.3)-(-0.25) | 2 |
| | 0.3-0.35 (-0.35)-(-0.3) | 1 |
| | 0.35 or more less than (-0.35) | 0 |
| Resistance (Y27) | (-30000) -30000 | 5 |
| | 30000-50000 (-50000)-(-30000) | 4 |
| | 50000-80000 (-80000)-(-50000) | 3 |
| | 80000-100000 (-10000)-(-80000) | 2 |
| | 100000-120000 (-120000)-(-100000) | 1 |
| | 120000 or more less than (-120000) (-120000) | 0 |

With respect to the answers 1-15 (Z231-Z245) to the questionnaire of the detailed test items which belong to the vitality (Y23), in the same manner as scoring of the answers 1-20 (Z211-Z220) to the questionnaire of the detailed test items which belong to the tranquilizing status (Y21), a total value is calculated as whole answers, then, the total value is scored in accordance with the scoring criteria corresponding to Table 7. In principle, numeric conversion is carried out by the answering number as it is in accordance with Table 5. However, regarding the answer listed as "reverse" in the remarks column of Table 5, there is a difference in that numeric conversion is carried out by {3- (answering number)} since either of "yes" or "no" for each of the answers 1-15 (Z231-Z245) to the questionnaire of the detailed test items which belong to the vitality (Y23) is selected by the examinee.

The amount of the beta-endorphin (Z251) of the examinee which belongs to the happiness (Y25) largely depends on an examinee's mental status. For this reason, in this embodiment, when the examinee undergoes assessment of his/her substantial age FF for the first time, thebeta-endorphin (Z251) is uniformly scored as "4" inprinciple. In a case that the examinee undergoes assessment of the substantial age FF second time or later (or in a case that the examinee undergoes assessment of the substantial age FF for the first time but the FF information providing site 10 has data of the past amount of beta-endorphin of the examinee), a percentage of the present amount of beta-endorphin to the former amount of beta-endorphin is calculated, and scoring for the beta-endorphin (Z251) is carried out in accordance with Table 7.

Because the more the detailed test item of the examineebecomes distant from a predetermined scope, the lower the scored point becomes with respect to both the adaptability in chaotic analysis (Z261) and the resistance in chaotic analysis (Z271), these two detailed test items for the examinee are scored by each degree of diverge in accordance with Table 7.

Scoring of detailed test items Z3j(k) belonging to the physical activity X3 is different from scoring of the detailed test items belonging to the kinestheticactivity X1 and the mental activity X2. namely, with respect to the peripheral blood (Y31), the lipids (Y32), the liver function (Y33), the gout (Y34) and the diabetes (Y35), if each of the detailed test items Z3j (k) falls outside the standard scope listed in the remarks column in Table 3, one point is deduced from a maximum point of 5 and the point after deducing becomes a scored point. Incidentally, in the remarks column of Table 3, the upper and lower portions respectively express a male and a female, and a right side thereof shows a unit of each of the detailed test items. For example, in a case that the examinee is a male, hemoglobin (Z311) is 12 g/deciliter, hematocrit (Z312) is 38%, erythrocyte (Z313) is 4,000,000/microliter, leukocyte (Z314) is 5,250/microliter, blood platelet is 200,000/microliter, because two of the hemoglobin (Z311) and hematocrit (Z312) fall outside the standard scopes, the scored point becomes 5-2=3. Incidentally, as obvious from Table 7, the scored point with respect to the peripheral blood (Y31) ranges 0-5, the lipids (Y32) ranges 1-5, the liver function (Y33) ranges 2-5, the gout (Y34) and diabetes (Y35) range 4-5.

Each of scored points for the percent body fat (Z361) and the obesity index (Z362) belonging to the body composition (Y36) is calculated by deducing one point from a maximum point of 5 in the same manner as the peripheral blood (Y31). Table 8 below shows scoring criteria for the baPWV (Z381-Z384) belonging to the vascular sclerosis (Y38) and the periphery area (Z391) belonging to the equilibrium function (Y39). Incidentally, because there are four detailed test items in the baPWV (Z381-Z384), the worst item (distant from the standard) is used in scoring in this embodiment.

Table 8

| Activity Sub-item | Measured Value | Scored Point |
|---|---|---|
| Bone Density (Y37) | 100% or more | 5 |
| | 80%-99.9% | 4 |
| | 75%-79.98 | 3 |
| | 70%74.9% | 2 |
| | 65%-69.9% | 1 |
| | less than 65% | 0 |
| Vascular Sclerosis (Y38) | +1 sigma or more | 5 |
| | median-(+1 sigma) | 4 |
| | (-1 sigma)-median | 3 |
| | (-2 sigma)-(-1 sigma) | 2 |
| | less than (-2 sigma) | 1 |

Table 8 (continued)

| Activity Sub-item | Measured Value | Scored Point |
|---|---|---|
| Equilibrium Function (Y39) | less than 3.0 cm$^2$ | 5 |
| | 3.0 cm$^2$-3.5 cm$^2$ | 4 |
| | 3.6 cm$^2$-4.5cm$^2$ | 3 |
| | 4.6 cm$^2$ or more | 2 |

As stated above, all the detailed test items Zijk belonging to the kinesthetic activity X1, the mental activity X2 and physical activity X3 are scored by points at a range of from 0 to 5, and the results of assessment or evaluation (scored points) become the activity sub-items Yij. Accordingly, each of the activity sub-items Yij is scored at a range of from 0 to 5. However, the grasping power (Z111-2 and the strength of back muscle (Z113) are integrated into the muscular power (Y11) through an integration step A (See Fig. 2). Namely, it was a subject to weight the two detailed test items (Z111-3) in the process of development of the age assessment program, a weight coefficient will to the grasping power (Z111-2) and a weight coefficient w112 were determined in accordance with a technical view made by the plurality of the specialists. The weight coefficient will and the weight coefficient w112 have a relationship that a sum of these coefficients is 1. The weight coefficient will and the weight coefficient w112 were set to w111= w112=0.5 in the age assessment program since the two detailed test items were equally important according to the technical view made by the specialists.

Incidentally, in the development process cf the integration step A, studies were made based on (A) Is the data classified by a male and a female as well as an age (group) sufficient?; and (B) Is there an influence of aging? Namely, with respect to the data classified by a male and a female as well as an age (group), tests were made as to a specific distribution (ex. normal distribution) with a predetermined significance level at every sex and age (group) , and then it was determined that the data were sufficient when the specific distribution was recognized at every sex and age (group). With respect to the influence of aging, a regression line regarding an average of each age (group) was found, and then it was determined that there was the influence of aging when the gradient of the regression line was smaller than a predetermined threshold value.

As shown in Fig. 2, each of the activity sub-items Yij was integrated into the activity items Xi through an integration step B in the process of development. As shown in Tables 1 to 3, a simple total of the kinesthetic activity X1, the mental activity X2 and the physical activity X3 ranges from 0 to 25 since each of them has the five activity sub-items Yij. Incidentally, since the peripheral blood (Y31), the lipids (Y32), the liver function (Y33), the gout (Y34) and the diabetes (Y35) of Table 3 are integrated into one group as stated above, scoring in a range of from 0 to 5 is carried out again as a group in which the five activity sub-items Y31-Y35 are integrated as stated later.

Here, an integration concept for the integration step B (See Fig. 2) which is one of the features of the present invention will be explained. For example, in a case in which the five sub-items Y11-Y15 are integrated into the kinesthetic activity X1 as shown in Table 1, how to weight with respect to each of the activity sub-items Y11-Y15 became a problem to be solved. In the present embodiment, analytic hierarchy process (AHP), which is announced in 1971 by Prof. Thomas L. Saaty of Pittsburgh Univ., was utilized to define weight coefficients qij for integrating the activity sub-items Yij into the activity items Xi.

In the process of the integration step B, first, Inventors asked the specialists to answer to weighting for determining the weight coefficients of the activity sub-items Yij to the activity items Xi. Namely, when the number of the activity sub-items Yij is n, Inventors asked the specialists about which is more important in determining the activity items Xi, an activity sub-item Yik or an activity sub-item Yil? with respect to the all pair {n(n-1)/2} in the activity sub-items, to answer with a selection of any one of the followingnine, stages; (1) the activity sub-item Yikis extremely important, (2) the activity sub-item Yik is very important, (3) the activity sub-item Yik is considerably important, (4) the activity sub-item Yik is slightly important, (5) the activity sub-items Yik and Yil are equally important, (6) the activity-sub-item Yil is slightly important, (7) the activity sub-item Yil is considerably important, (8) the activity sub-item Yil is very important, (9) the activity sub-item Yil is extremely important.

Next, based on the answer of the specialists, a weight akl was set in a manner that the weight akl=9 when the activity sub-item Yik was extremely important, the weight akl=7 when the activity sub-item Yik was very important, the weight akl=5 when the activity sub-item Yik is considerably important, the weight akl=3 when the activity sub-item Yik is slightly important, and the weight akl=1 when the activity sub-items Yik and Yil are equally important. In cases that the activity sub-items Yij were more important, each value of weight alk was set in the same manner as the value of weigh akl in accordance with the degree of importance. Incidentally, it was defined that alk=(1/akl).

In these circumstances, a pair comparison matrix Ai=[akl] and a weight vector qi=(qi1, qi2,..., qin) are defined. By finding an eigenvalue vector qi which satisfies an eigenvalue problem of (Ai - n · I)qi=0, weight coefficients were set based on the found eigenvalue vector qi. Incidentally, a sum of the weight coefficients becomes 1. ($\Sigma$qij=1 at every activity item Xi)

With respect to the integration group consisting of the peripheral blood (Y31), the lipids (Y32), the liver function (Y33), the gout (Y34) and the diabetes (Y3S) of Table 3, each of weight coefficients u3n (n=1 to 5) was set so that a sum of the weight coefficients of the five activity sub-items Y31 to Y35 becomes 1. Accordingly, the integration group is scored in a range of from 0 to 5 by calculating (a scored point) = (u31 x the peripheral blood (Y31)} + (u32 x the lipids (Y32)} + (u33 x the liver function (Y33)} + (u34 $\times$ the gout (Y34)} + (u35 x the diabetes (Y35)}.

Then, in order to prepare an integration step C below, in the integration step B, calculation (conversion) from each of the activity sub-items Yij to an age difference value $\Delta$yij which expres ses a degree of age divergence from an actual age of the examinee is carried out in accordance with Table 9 below.

Table 9

| Scored Point | Age difference Value |
|:---:|:---:|
| 5 | 1 |
| 4 | 0 |
| 3 | -1 |
| 2 | -2 |
| 1 | -3 |
| 0 | -4 |

For example, in a case that a scoring point of the muscular power (X11) of the examinee is 5, the scored point is changed into an age difference value $\Delta$yll = "1", and a scoring point of the muscular power (Y11) is 2, it is changed into an age difference value $\Delta$yll = "-2" in accordance with Table 9. As stated above, in the development process of the integration step A, studies were made on (A) Is the data classified by a male and a female as well as an age (group) sufficient ?; and (B) Is there an influence of aging? while, in the development process of the integration step B, tests for a relationship among the scored points of the activity sub-items Yij, the detailed test items Zijk and the age difference values $\Delta$yij were carried out by appropriately using a plurality of integration techniques base upon the studies on the above (A) and (B); (1) an integration technique based on a statistical average value was used when the above (A) and (B) were affirmed, (2) an integration technique based on a statistical distribution was used when the above (A) was affirmed but the above (B) was denied, (3) an integration technique based on a hypothetical distribution was used when the above (A) and (B) were denied.

The test results of the above integration techniques (1) to (3) of the present embodiment are briefed as follows. (1) In the integration technique based on a statistical average value, when an age in which the value of each detailed test item falls on an average value is assumed as an average age for the scored point of the detailed test item, and when a degree of age divergence from an actual age of the examinee (examinee C) with respect to the detailed test item is an age difference value $\Delta$zijk, a test is carried out for each age difference value $\Delta$zijk as to whether or not the age difference value $\Delta$yij shown in Table 9 becomes equal to a sum obtained by multiplying the age difference values $\Delta$zijk of each of the detailed test items Zijk and each of the weight coefficients wijk together. The integration technique (1) had a significance standard of 93% or more. (2) In the integration technique based on a statistical distribution, a test was carried out by assuming that the distribution is a normal distribution in the same manner as the integration technique (1). The integration technique (2) had a significance standard of 90% or more. (3) In the integration technique based on a hypothetical distribution which has a distribution other than a normal distribution such as a "$x^2$" distribution, a "t" distribution, or the like, an appropriate distribution was determined with reference to opinions of the specialists, and dividing threshold values $\theta$ were set in accordance with degrees of divergence in the hypothetical distribution, then, a test was carried out in the same manner as the integration technique (1). The integration technique (3) had a significance standard of 87% or more. Accordingly, there is a correlation between scoring of the activity sub-items Yij shown in Table 9 and the age difference values $\Delta$ yij under the predetermined significance standard.

Further, in the integration step B, each of age difference values Axi of the activity items Xi is calculated as a sum obtained by the age difference values $\Delta$yij of the activity sub-items Yij belonging to the activity items Xi and the weight coefficients qij together in accordance with the following Formula (2).

$$\Delta xi = \sum_{j=1}^{ri} qij \bullet \Delta yij \qquad\qquad \cdot\cdot\cdot \quad (2)$$

Next, in an integration step C (See Fig. 3), each of the age difference values Δxi of the activity items Xi is integrated into an age difference value Δf which is an indicator of the substantial age FF. In other words, the age difference value Δf means a degree of age divergence, which reflects many-sides of the kinesthetic activity, the mental activity and the physical activity, from an actual age of the examinee. Incidentally, the age difference value Δf has a decimal in principle. By utilizing the above stated AHP in the same manner as the integration step B, an eigenvalue vector pi was found to obtain weight coefficients pi, after questions (three questions due to three activity items) to the specialists were made, nine stage weighting to answers made by the specialists was determined, and a pair comparison matrix was defined.

$$\Delta f = p1 \bullet \Delta x1 + \mu2 \bullet \Delta x2 + \mu3 \bullet \Delta x3 \qquad\qquad (3)$$

Further, in the integration step C, the substantial age FF of the examinee is calculated according to the following Formula (4). In Formula (4), the substantial age FF is calculated by subtracting the age difference value Δ from the actual age "a" with a relationship of Table 9. However, the substantial age FF may be calculated by adding the age difference value Δf to the actual age "a" by changing the signs in Table 9. Incidentally, when the substantial age has a decimal, the decimal is omitted so that the examinee understands his/her actual age easily.

$$FF = a - \Delta f \qquad\qquad (4)$$

(Operation)

Next, operation of the age assessment system of the present embodiment will be explained.

First, an operator of the terminal 4, 5 activates input software installed to the terminal 4, 5 in advance in order to input bibliographical items and the detailed test items Zijk of the examinee. The input software is coded by a language such as C++, VB, or the like, and check buttons to which predetermined programs are allocated respectively are disposed at an image screen thereof.

The bibliographical items include a full name, age, sex, address, telephone number, mobilephonenumber, height, weight, unique ID number of the examinee and the examined date. When an operator clicks a check button, the CPU of the terminal 4, 5 (hereinafter called "terminal CPU") checks as to whether or not there are input omissions and wrong numbers in digits in accordance with program of the input software. When there are input omissions or wrong numbers in digits, the terminal CPU displays that there are input omissions or wrong numbers in digits and waits until (re) input is carried out by an operator. When input is carries out, the terminal CPU checks again as to whether or not there are inputting omissions and wrong number of digits, and if there are no input omissions and wrong numbers in digits regarding the bibliographical -tems, all of the bibliographical items are memorized in the RAM. Incidentally, the mobile phone number is handled as an optional input item.

When an operator clicks other check button, the terminal CPU determines as to whether or not all data of the detailed test items Zijk belonging to three or more activity sub-items Yij which belong to each of the activity items Xi are filled (not null). As stated above, since the age assessment program assesses the substantial age of the examinee from many sides, it is preferable that there exist all data of the detailed test items Zijk which support three or more activity sub-items Yij in order to secure proper assessment. If there are all data of the detailed test items Zijk belonging to three or more activity sub-items Yij, the terminal CPU determines that inputting of the bibliographical items were appropriately carried out, then it judges as to whether or not there are outliers (abnormal values) in the data of the inputted detailed test items Zijk. When there are outliers, the terminal CPU displays that there are outliers and wait until input (revision) is carried out again by an operator. when input is carried out again, the terminal CPU judges again as to whether or not there are outliers, and it waits until revision is carried out. The terminal CPU repeats the same judgment and waiting until there are no outliers in the data of the inputted detailed test items Zij k. When there are no outliers, the terminal CPU memorizes all of the data of the inputted detailed test items Zijk in the RAM as proper data, and displays that it is ready to transmit the data to the FF information providing site 10. The diagnostic criteria for the outliers are incorporated in program of the input software in advance by reflecting the studied results of the specialists.

On the other hand, when all of the data of the detailed test items Zijk which support one activity sub-item Yij are not inputted, or, when the data of the detailed test items Zijk which support a part of activity sub-items Yij are inputted but the number of the activity sub-items Yij supported by the detailed test items Zijk perfectly is less than three, the terminal CPU displays the input defects and waits until input is carried cut by an operator. When input is carried, the terminal CPU judges input defects again in the same manner. When there exist all of the data of the detailed test items Zijk which belong to three or more activity sub-items Yij, it judges as to the above stated outliers. When no outliers exist in the inputted detailed test items Zijk, the terminal CPU memorizes all of the data of the inputted detailed test items Zijk in the RAM as proper data, and displays that it is ready to transmit the data to the FF information providing site 10.

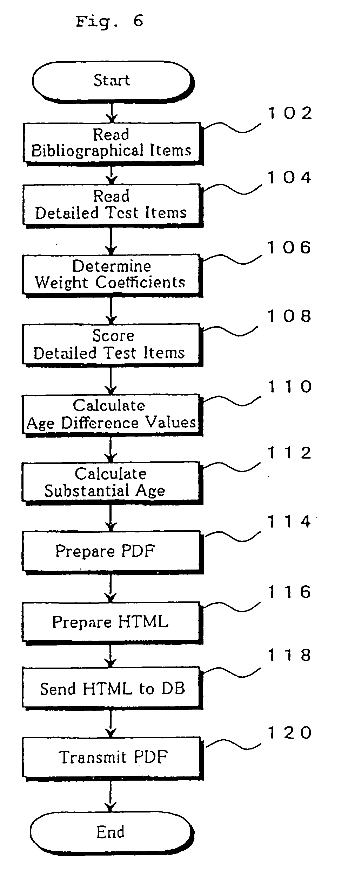

On the image screen of the input software, a transmission button to which predetermined program is allocated is also disposed. An operator can transmit the proper data of the detailed test items Zijk to the FF information providing site 10 by clicking the transmission button. The CPU of the www server 13 which receives the data from the terminal 4, 5 stores the received data into DB server 14, and executes, in accordance with the above stated age assessment program, an age assessment routine shown in Fig. 6.

In the age assessment routine, first, in step 102, the CPU reads out the bibliographical items stored in the DB server 14, then in the next step 104, reads out the detailed test items Zijk and carries out preprocessing such as the arithmetic mean between the right hand grasping power Z111 and the left hand grasping power Z112, the calculation for obtaining the total values with respect to the questionnaire. Further, in the step 104, the CPU judges as to whether or not there are past bibliographical items for the examinee of the same ID number in the DB server 14. If there are not past bibliographical items, the CPU judges as to whether there is data with respect to the amount of the beta-endorphin of the examinee in a predetermined area of the bibliographical items read out from the DB server 14. If the judgment is negative, the scoring point forthehappiness (Y25) is fixed to "4". If the judgment is affirmative, the CPU calculates a percentage of the present amount of the beta-endorphin (Z251) to a previous (former) amount of the beta-endorphin (Z251) by regarding the amount of the beta-endorphin in the predetermined area as the previous amount of the beta-endorphin (Z251). On the other hand, if there are past bibliographical items for the examine of the same ID number in the DB server 14, the CPU judges as to whether there is previous data with respect to the amount of the beta-endorphin of the examinee of the same ID number in the bibliographical items. If the judgment is affirmative, the CPU reads out the previous data of the amount of the beta-endorphin (Z251) and calculates a percentage of the present amount of the beta-endorphin (Z251) to a previous amount of the beta-endorphin (Z251). If the judgment is negative, the scoring point for the happiness (Y25) is fixed to "4".

In the next step 106, the weight coefficients qij are determined. As stated above, the weight coefficients qij were digitized assuming that there are five activity sub-items Yij which belong to one activity item Xi. However, all of the five activity sub-items Yij can not be obtained necessarily like in a case that there is not data of the detailed test item zijk for the periphery area (Z391), for example, due to that there is not the centroid fluctuation measuring instrument. In the present embodiment, as stated above, each of the activity items Xi has three or more activity sub-items Yij . For example, if three activity sub-items Yij belong to one activity item Xi, correction is carried out from the weight coefficients for the five activity sub-item Yij to weight coefficients qij of three activity sub-items Yij. In a case that the five weight coefficients qij are, for example, qi1=0.235, qi2=0.117, qi3=0.315, qi4=0.188 and qi5=0.145, and there are no detailed test items Zijk of activity sub-items corresponding to the weight coefficients qi2 and qi5, corrected weight coefficients qi1, qi3 and qi4 are calculated by multiplying each of the weight coefficients qi1, qi3 and qi4 and (1/ (qi1+qi1+qi4) ) together so that a sum of the weight coefficients qi1, qi3 and qi4 becomes 1. The (corrected) weight coefficients are memorized in the RAM.

In the next step 108, with respect to each of the activity sub-items Yij, scoring in the range of from 0 to 5 is carried out in accordance with the scoring criteria shown in Tables 3, 6, 7, 8 and the like, then in step 110, the age difference value Δyij for each of the activity sub-items Yij is calculated in accordance with the conversion table shown in Table 9. In the next step 112, the weight coefficients qij memorized in the step 106 are read out and the substantial age FF is calculated in accordance with Formulae (2) to (4). The substantial age FF is also memorized in the RAM.

In step 114, the CPU prepares PDF having a predetermined format in order to report the assessment results to a client (terminal 4, or 5) as the FF information. The FF information includes the bibliographical items, the substantial age FF, each activity item Xi, each activity sub-item Yij and scored point thereof, the detailed test items Zijk, and the like. Incidentally, when there are portions where the scored point is 2 or less, asterisks are indicated to the portions for the examinee's reference.

Next, in step 116, the CPU judges as to whether or not there is a mobile phone number in the bibliographical items. If the judgment is negative, the routine proceed to step 118. If the judgment is affirmative, HTML which is allowed to access with a mobile phone is prepared so that the examinee can access the FF information providing site 10 with his/her mobile phone. In the HTML, name, actual age, substantial age FF, each activity item Xi, each activity sub-item Yij and scoring point thereof, which constitute a part of the FF information, are listed. Incidentally, if the FF information providing site 10 receives access from a mobile phone, the FF information providing site 10 discloses the HTML to the person who accessed from a mobile phone, for example, only when the name, the ID number and the examined date are identical with the data stored in advance to the DB server 14 in view of information protection.

In step 118, the calculation results calculated up to the step 112 and the HTML prepared in the step 116 are sent to the DB server 14 so as the DB server 14 to memorize the data. Then, in step 120, the CPU transmits the PDFpreperedin the step 114 to the terminal 4 or 5, and the age assessment routine ends.

The DB server 14 deletes HTML which passed a predetermined period of time (ex. 7 days) to lighten its load. The DB server 14 increases the number of data which constitute the population for each of the detailed test items Zij k by adding newly added data for each of the detailed test items Zijk at every predetermined period of time (ex. 10 days) so that the specialists can review (renew) the statistical distribution shown in Table 5 and the like at every predetermined period of time, and controls the number N of samples which constitutes the each of the detailed test items Zijk and the renewal date. Incidentally, the charging computer 15 calculates the charging fees by referring to the data stored in the DE server 14 at every predetermined period of time to issue bills.

(Effects and the like)

Next, effects and the like of the age assessment system of the present embodiment will be explained.

As statedabove, since the age assessment systemof the present embodiment assesses the substantial age FF from many sides according to the plurality of detailed test items Zijk through integration steps A to C for integrating the detailed test items Zijk into the kinesthetic activity X1, the mental activity X2 and the physical activity X3, and assesses the substantial age FF totally from three different viewpoints of the kinesthetic activity X1, the mental activity X2 and the physical activity X3, the age assessment system can reflects the degree of health to the substantial age from many sides totally. Accordingly, the age assessment system can express the substantial age of the examinee comprehensively comparing with the prior art which could express only either one of the kinesthetic activity, the mental activity and the physical activity, or which could express a few other assessment elements thereto additionally. For this reason, not only an examinee who has some trouble on kinesthetic performance, metal status or body but also a healthy examinee can grasp his/her degree of health. Further, since the age assessment system of the present embodiment assesses the examinee with an age, the examine who has little medical knowledge can easily understand his/her degree of health. Furthermore, since the age assessment system of the present embodiment transmits the FF information with the format of FDF to a client, it can show information having a hierarchic structure with respect to the substantial age, the activity items Xi, the activity sub-items Yij and the detailed test items Zijkto the examinee as it is (See Fig. 2) . Besides, since the asterisks are indicated to the portions in which the examinee has trouble in his/her health and since the age difference value $\Delta$xi is expressed to each of the activity items Xi and the scored point is expressed to each of the activity sub-items Yij, the examinee who has some trouble on kinesthetic performance, metal status or body can grasp the point(s) in trouble clearly. Accordingly, the FF information can contributes to the examinee's preservation to some disease at an early stage.

Moreover, the age assessment systemof the present embodiment is constituted to add new data for each of the detailed test items in order to renew the size of the population for each of the detailed test items Zijk at every predetermined period of time so that the specialists can review the statistical distribution, the age assessment system has developmental possibilities that it can assess the substantial age FF more precisely in accordance with an increase in the number of the examinees. In this case, since Formulae (2) and (3) have the weight coefficients, it stand a chance that the assessment of the substantial age FF can be improved by changing the weight coefficients dynamically in accordance with the judgment made by the specialists. Further, the age assessment system of the present embodiment can add new detailed test items Zijk as shown in Fig. 2. Since medical treatment and medical appliances are making rapidprogress and the age assessment systemcan fetch such new detailed test items Zijk flexibly, the age assessment system can assess such new detailed test items Zijk rapidly from statistical distributions thereof. Therefore, the age assessment system of the present embodiment has possibilities in progress and flexibility.

Incidentally, in the above embodiment, an example in which the integration steps A and B with respect to the kinesthetic activity X1, the mental activity X2 and the physical activity X3 are processed in the steps 108, 110 of Fig. 6 all at once was shown for convenience of explanation. However, since it is necessary to obtain the age difference value for each of the kinesthetic activity X1, the mental activity X2 and the physical activity X3 in order to calculate the substantial age FF, the substantial age FF may be calculated, for example, after the age difference value $\Delta$x1 for the kinesthetic activity X1, the age difference value $\Delta$x2 for the mental activity X2, and the age difference value $\Delta$x3 for the physical activity X3 are calculated sequentially.

Further, in the present embodiment, an example where each of the activity sub-items Yij is scored by point with the six stage evaluation ranging from 0 to 5 and the scored points are converted to each of the age difference values $\Delta$yij was explained. However, age difference value $\Delta$zijk can be calculated at every detailed test items Zijk in place of the six stage evaluation ranging from 0 to 5, and in the step A, the calculated age difference values $\Delta$zijk may be integrated into each of the age difference values $\Delta$yij of the activity sub-items $\Delta$Yij to integrate the age difference values Δyij into each of the age difference values Δxi in the step B. In this way, the detailed test items Zijk are integrated to the substantial age FF only by the age difference values from the integration steps AtoC. Accordingly, Inventors understand that the terms "kinesthetic performance scoring criteria", "mental state scoring criteria" and "physical information scoring criteria" in the claims hereinafter claimed include a concept of calculating the age difference values Δzijk instead of the six stage evaluation, or, calculation of the age difference values Δzijk is at least equivalent to the six stage evaluation.

Furthermore, in the present embodiment, an example the WWW server 13 and the DB server 14 separately functions as an age assessment apparatus was shown. However, it goes without saying that the data to be memorized in the D3 server 14 may be stored in a hard disc of the WWW server 13. Besides, in the present embodiment, an example in which an age (group) is represented by every ten years and the scoring point for the six stage evaluation is expressed by an integral number was shown for simplification of explanation. However, the present invention is not limited to the same. For example, the scoring criteria may be made at ages of two or three yeas and they may be made at 0.5 point in order to enhance precision for the substantial age FF of the examinee.

## Claims

1. An age assessment: apparatus that assesses a substantial age of an examinee based upon biological information of the examinee, comprising:

   a first calculator for scoring a plurality of kinesthetic performance information of the examinee in accordance with predetermined kinesthetic performance scoring criteria and calculating a kinesthetic age difference value which expresses a degree of age divergence from an actual age of the examinee with respect to kinesthetic activity which expresses an active degree of a kinesthetic performance of the examinee;
   a second calculator for scoring a plurality of mental state information of the examinee in accordance with predetermined mental state scoring criteria and calculating a mental age difference value which expresses a degree of age divergence from an actual age of the examinee with respect to mental activity which expresses an active decree of a mental state of the examinee;
   a third calculator for scoring a plurality of physical information of the examinee in accordance with predetermined physical information scoring criteria and calculating a physical age difference value which expresses a degree of age divergence from an actual age of the examinee with respect to physical activity which expresses an active degree of a body of the examinee; and
   an age calculator for calculating a substantial age of the examinee, in which the kinesthetic activity, the mental activity and the physical activity are integrated, based upon an actual age of the examinee and each of the age difference values calculated by the first to third calculators.

2. An age assessment apparatus according to claim 1, wherein at least one of the kinesthetic performance scoring criteria, the mental state scoring criteria and the physical information scoring criteria is criteria classified by a male and a female, and an age or an age group.

3. An age assessment apparatus according to claim 2, wherein the kinestheticperformance scoring criteria, themental state scoring criteria and the physical information scoring criteria are scoring criteria corresponding to a distribution scope of statistical distribution of a large number of examinees.

4. An age assessment apparatus according to claim 1, wherein the age calculator adds/subtracts a sum obtained by multiplying each of the age difference values calculated by the first to third calculators and each of weight coefficients corresponding to the each of the age difference values together to/from the actual age of the examinee to calculate the substantial age of the examinee.

5. An age assessment apparatus according to claim 4, wherein the weight coefficients constitute an eigenvector which is a solution of an eigenvalue problem including a pair comparison matrix obtained by analytic hierarchy process.

6. An age assessment apparatus according to claim 5, wherein a sum of the each of the weight coefficients is 1.

7. An age assessment apparatus according to claim 1, wherein the first to third calculators score the plurality of kinesthetic performance information in accordance with the kinesthetic performance scoring criteria, the plurality of mental state information in accordance with the mental state scoring criteria, and the plurality of physical information in accordance with the physical information scoring criteria by points, respectively, then, convert the scored points to the age difference values which express the degrees of the age divergence from the actual ages of the examinee with respect to the plurality of kinesthetic performance information, the plurality of mental state information and the plurality of physical information respectively, and calculate sums obtained by multiplying each of the age difference values and each of weight coefficients which correspond to the each of the age difference values and which are solutions of eigenvalue problems including pair comparison matrixes obtained by analytic hierarchy process together as the kinesthetic age difference value, the mental age difference value and the physical age difference value, respectively.

8. An age assessment apparatus according to claim 1, wherein the physical information includes at least three pieces of information among information as to blood, information as to body composition including obesity index, information as to bone density, information as to vascular sclerosis and information as to equilibrium function of the examinee.

9. An age assessment apparatus according to claim 1, wherein the kinestheticperformance information includes at least three pieces of information among information as to muscular power, information as to muscular endurance strength, information as to instantaneous power, information as to flexibility and information as to quickness of the examinee.

10. An age assessment apparatus according to claim 1, wherein the mental state information includes at least three pieces of information as to vitality, information as to tranquilizing status, information as to beta-endorphin in blood, information as to adaptability in chaotic analysis and resistance in chaotic analysis of the examinee.

11. An age assessment method for assessing a substantial age of an examinee based upon biological information of the examinee, comprising:

a first calculating step for scoring a plurality of kinesthetic performance information of the examinee in accordance with predetermined kinesthetic performance scoring criteria and calculating a kinesthetic age difference value which expresses a degree of age divergence from an actual age of the examinee with respect to kinesthetic activity which expresses an active degree of a kinesthetic performance of the examinee;
a second calculating step for scoring a plurality of mental state information of the examinee in accordance with predetermined mental state scoring criteria and calculating a mental age difference value which expresses a degree of age divergence from an actual age of the examinee with respect to mental activity which expresses an active degree of a mental state of the examinee;
a third calculating step for scoring a plurality of physical information of the examinee in accordance with predetermined physical information scoring criteria andcalculating aphysical age difference value which expresses a degree of age divergence from an actual age of the examinee with respect to physical activity which expresses an active degree of a body of the examinee; and
an age calculating step for calculating a substantial age of the examinee, in which the kinesthetic activity, the mental activity and the physical activity are integrated, based upon an actual age of the examinee and each of the age difference values calculated by the first to third calculating steps.

12. An age assessment method according to claim 11, wherein at least one of the kinesthetic performance scoring criteria, the mental state scoring criteria and the physical information scoring criteria is criteria classified by a male and a female, and an age or an age group.

13. An age assessment method according to claim 12, wherein the kinestheticperformance scoringcriteria, thementalstatescoring criteria and the physical information scoring criteria are scoring criteria corresponding to a distribution scope of statistical distribution of a large number of examinees.

14. An age assessment method according to claim 11, wherein in the age calculating step, a sum obtained by multiplying each of the age difference values calculated by the first to third calculating steps and each of weight coefficients corresponding to the each of the age difference values together is added/subtracted to/from the actual age of the examinee to calculate the substantial age of the examinee.

15. An age assessment method according to claim 14, wherein the weight coefficients constitute an eigenvector which is a solution of an eigenvalue problem including a pair comparison matrix obtained by analytic hierarchy process.

**16.** An age assessment method according to claim 15, wherein a sum of the each of the weight coefficients is 1.

**17.** An age assessment method according to claim 11, wherein in the first to third calculating steps, the plurality of kinesthetic performance information are scored in accordance with the kinesthetic performance scoring criteria, the plurality of mental state information in accordance with the mental state scoring criteria, and the plurality of physical information in accordance with the physical information scoring criteria by points, respectively, then, the scored points are converted to the age difference values which express the degrees of the age divergence from the actual ages of the examinee with respect to the plurality of kinesthetic performance information, the plurality of mental state information and the plurality of physical information respectively, and sums obtained by multiplying each of the age difference values and each of weight coefficients which correspond to the each of the age difference values and which are solutions of eigenvalue problems including pair comparison matrixes obtained by analytic hierarchy process together are calculated as the kinesthetic age difference value, the mental age difference value and the physical age difference value, respectively.

**18.** An age assessment method according to claim 11, wherein the physical information includes at least three pieces of information among information as to blood, information as to body composition including obesity index, information as to bone density, information as to vascular sclerosis and information as to equilibrium function of the examinee.

**19.** An age assessment method according to claim 11, wherein the kinestheticperformance information includes at least three pieces of information among information as to muscular power, information as to muscular endurance strength, information as to instantaneous power, information as to flexibility and information as to quickness of the examinee.

**20.** An age assessment method according to claim 11, wherein the mental state information includes at least three pieces of information as to vitality, information as to tranquilizing status, information as to beta-endorphin in blood, information as to adaptability in chaotic analysis and resistance in chaotic analysis of the examinee.

Fig. 1

Fig. 2

EP 1 533 740 A2

Fig. 3

Fig. 4

Fig. 5A

Fig. 5B

Fig. 6

```
            ┌──────────────┐
            │    Start     │
            └──────┬───────┘
                   ↓
        ┌────────────────────┐
        │       Read         │  102
        │ Bibliographical Items │
        └────────┬───────────┘
                 ↓
        ┌────────────────────┐
        │       Read         │  104
        │ Detailed Test Items │
        └────────┬───────────┘
                 ↓
        ┌────────────────────┐
        │     Determine      │  106
        │ Weight Coefficients │
        └────────┬───────────┘
                 ↓
        ┌────────────────────┐
        │      Score         │  108
        │ Detailed Test Items │
        └────────┬───────────┘
                 ↓
        ┌────────────────────┐
        │    Calculate       │  110
        │ Age Difference Values │
        └────────┬───────────┘
                 ↓
        ┌────────────────────┐
        │    Calculate       │  112
        │  Substantial Age   │
        └────────┬───────────┘
                 ↓
        ┌────────────────────┐
        │    Prepare PDF     │  114
        └────────┬───────────┘
                 ↓
        ┌────────────────────┐
        │    Prepare HTML    │  116
        └────────┬───────────┘
                 ↓
        ┌────────────────────┐
        │  Send HTML to DB   │  118
        └────────┬───────────┘
                 ↓
        ┌────────────────────┐
        │    Transmit PDF    │  120
        └────────┬───────────┘
                 ↓
            ┌──────────────┐
            │     End      │
            └──────────────┘
```